(19) Europäisches Patentamt
European Patent Office
Office européen des brevets

(11) **EP 2 924 434 B1**

(12) **EUROPEAN PATENT SPECIFICATION**

(45) Date of publication and mention
of the grant of the patent:
**21.02.2018 Bulletin 2018/08**

(21) Application number: **15160044.2**

(22) Date of filing: **20.03.2015**

(51) Int Cl.:
**G01N 33/53** *(2006.01)*     **G01N 1/28** *(2006.01)*
**G01N 1/36** *(2006.01)*     **G01N 1/40** *(2006.01)*
**G01N 1/44** *(2006.01)*     **G01N 15/10** *(2006.01)*
**G01N 15/14** *(2006.01)*

(54) **CELL ANALYSIS METHOD**

ZELLANALYSEVERFAHREN

PROCÉDÉ D'ANALYSE DE CELLULE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2014 JP 2014069745**

(43) Date of publication of application:
**30.09.2015 Bulletin 2015/40**

(73) Proprietor: **SYSMEX CORPORATION
Kobe-shi,
Hyogo 651-0073 (JP)**

(72) Inventors:
- **Kiriyama, Maria
  Hyogo, 651-0073 (JP)**
- **Kubo, Takuya
  Hyogo, 651-0073 (JP)**

(74) Representative: **Hoffmann Eitle
Patent- und Rechtsanwälte PartmbB
Arabellastraße 30
81925 München (DE)**

(56) References cited:
- **WILLEM E CORVER ET AL: "High-resolution multi-parameter DNA flow cytometry enables detection of tumour and stromal cell subpopulations in paraffin-embedded tissues", THE JOURNAL OF PATHOLOGY, vol. 206, no. 2, 1 January 2005 (2005-01-01), pages 233-241, XP055194131, ISSN: 0022-3417, DOI: 10.1002/path.1765**
- **M.P.G. LEERS ET AL: "Heat pretreatment increases resolution in DNA flow cytometry of paraffin-embedded tumor tissue", CYTOMETRY, vol. 35, no. 3, 1 March 1999 (1999-03-01), pages 260-266, XP055194137, ISSN: 0196-4763, DOI: 10.1002/(SICI)1097-0320(19990301)35:3<260: :AID-CYTO9>3.0.CO;2-O**
- **Willem E. Corver ET AL: "High-Resolution Multiparameter DNA Flow Cytometry for the Detection and Sorting of Tumor and Stromal Subpopulations from Paraffin-Embedded Tissues" In: "Current Protocols in Cytometry", 1 January 2011 (2011-01-01), John Wiley & Sons, Inc., Hoboken, NJ, USA, XP055194143, ISBN: 978-0-47-114295-9 DOI: 10.1002/0471142956.cy0737s55, * pages 7.37.1-7.37.5, page 7.37.15 ***
- **Shigeki Namimatsu ET AL: "Reversing the Effects of Formalin Fixation with Citraconic Anhydride and Heat: A Universal Antigen Retrieval Method", Journal of Histochemistry and Cytochemistry, 1 January 2005 (2005-01-01), pages 3-11, XP055194171, DOI: 10.1369/jhc.4C6466.2005 Retrieved from the Internet: URL:http://www.jhc.org/cgi/content/abstract/53/1/3 [retrieved on 2015-06-02]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 924 434 B1

SEQUENCE LISTING

<110> SYSMEX CORPORATION

<120> Method for analyzing cell

<130> 13-048JP

<160> 1

<170> PatentIn version 3.5

<210> 1
<211> 5
<212> PRT

<220>

<223> Artificial

<220>
<223> A sequence for collagenase substrate

<220>
<221> MISC_FEATURE
<222> (1)..(1)
<223> Xaa represents 4-phenyl-azobenzyl-oxycarbonyl-proline residue

<400> 1

```
Xaa Leu Gly Pro Arg
1               5
```

## Description

FIELD OF THE INVENTION

The invention relates to a cell analysis method. More specifically, the invention relates to a cell analysis method suitable for analyzing cells constituting a tumor tissue, a non-tumor diseased tissue, a normal tissue, or the like contained in a tissue specimen which is aldehyde-fixed and embedded in a water-insoluble embedding medium.

BACKGROUND

Conventionally, samples used in a population analysis on cell groups constituting a tissue such as a solid tumor tissue (tumor tissue), a non-tumor diseased tissue, or a normal tissue, have been obtained by isolating individual cells from a living tissue. However, such cells obtained from a living tissue are likely to differ from each other in the state, making the quality control difficult. Moreover, in the analysis, a long time is required when cells in a tissue are separate into individual cells. Hence, the original characteristics that the individual cells have only in a three-dimensional structure may change or disappear in some cases.

Accordingly, a method has been proposed in which after a tissue is formalin-fixed and paraffin-embedded (hereinafter referred to as "FFPE") to temporarily store the original characteristics of cells in a three-dimensional structure, the cells are separated into individual cells for analysis (see, for example, Non-patent Literature 1).

Non-patent Literature 1 discloses that when single cells are obtained from a FFPE tissue specimen in a cell analysis using a flow cytometer, a thin section of the FFPE tissue specimen is heated in a solution containing citric acid.

Non-patent Literature 1

Willem E Corver et al., "High-resolution multi-parameter DNA flow cytometry enables detection of tumour and stromal cell subpopulations in paraffin-embedded tissues," The Journal of Pathology, published online April 11, 2005 (http://interscience.wiley.com), Vol. 206, pp. 233-241

SUMMARY OF THE INVENTION

The scope of the invention is defined by the appended claims, and not by any statements within this summary.

An aspect of the present invention is a cell analysis method of analyzing, using a flow cytometer, cells obtained from a tissue specimen, which are aldehyde-fixed and embedded in a water-insoluble embedding medium. The method is characterized by including: (a) removing the embedding medium from a sample obtained from the tissue specimen which is aldehyde-fixed and embedded in the water-insoluble embedding medium, thereby obtaining the sample containing the tissue from which the embedding medium is removed; (b) heating the sample obtained in step (a) in the presence of a divalent carboxylic acid compound, thereby obtaining the heat-treated sample containing the tissue; (c) bringing the sample obtained in step (b) into contact with an enzyme with a cell dispersion activity to separate, into individual cells, cells contained in the sample obtained in step (b), thereby obtaining the sample containing the individual cells dispersed in a solvent; (d) obtaining, using the flow cytometer, optical information on the cells in the sample obtained in step (c); and (e) analyzing the cells based on the optical information obtained in step (d).

BRIEF DESCRIPTION OF THE DRAWINGS

Fig. 1 is a graph for illustrating the results of examining relative dispersion efficiencies obtained by using test samples of Examples 1 to 7 and Comparative Examples 1 to 5.

Fig. 2 is a graph for illustrating the results of examining cell dispersion efficiencies obtained by using test samples of Example 8 and Comparative Example 6.

Fig. 3A is a scattergram obtained by using an aqueous solution of 0.1 mass % maleic acid (Example 9), Fig. 3B is a scattergram obtained by using an aqueous solution of 0.1 mass % citraconic acid (Example 10), Fig. 3C is a scattergram obtained by using an aqueous solution of 0.1 mass % citraconic anhydride (Example 11), and Fig. 3D is a scattergram obtained by using an aqueous solution of 0.1 mass % citric acid (Comparative Example 7).

EMBODIMENTS

A cell analysis method according to an embodiment is a cell analysis method of analyzing, using a flow cytometer, cells obtained from a tissue specimen that is aldehyde-fixed and embedded in a water-insoluble embedding medium, the method including: (a) removing the embedding medium from a sample obtained from the tissue specimen which is aldehyde-fixed and embedded in the water-insoluble embedding medium, thereby obtaining the sample containing the tissue from which the embedding medium is removed; (b) heating the sample obtained in step (a) in the presence of a divalent carboxylic acid compound, thereby obtaining the heat-treated sample containing the tissue; (c) bringing the sample obtained in step (b) into contact with an enzyme having a cell dispersion activity to separate the tissue into individual cells, cells contained in the sample obtained in step (b), thereby obtaining the sample containing the individual cells dispersed in a solvent; (d) obtaining, using the flow cytometer, optical information of the cells in the sample obtained in step (c); and (e) analyzing the cells based on the optical information obtained in step (d).

The inventors have found as described in Comparative Examples later that when cells obtained from a tissue specimen which is aldehyde-fixed and embedded in a water-insoluble embedding medium are analyzed using a flow cytometer, if the tissue specimen is heated in a solution containing citric acid after removal of the embedding medium from the tissue specimen, a large number of cell lumps not separated into individual cells remain in the cells analyzed using the flow cytometer. Then, the inventors have surprisingly found that subjecting the sample with the embedding medium having been removed not to the heat treatment in a solution containing citric acid but to a heat treatment in the presence of a divalent carboxylic acid compound makes it possible to separate and disperse individual cells into the solvent with higher dispersion efficiency.

The inventors have found as described in Comparative Examples later that when cells obtained from a tissue specimen which is aldehyde-fixed and embedded in a water-insoluble embedding medium are analyzed using a flow cytometer, if the tissue specimen is heated in a solution containing citric acid after removal of the embedding medium from the tissue specimen, a large number of cell lumps not separated into individual cells remain in the cells analyzed using the flow cytometer. Then, the inventors have found that subjecting the sample with the embedding medium having been removed not to the heat treatment in a solution containing citric acid but to a heat treatment in the presence of a divalent carboxylic acid compound makes it possible to separate and disperse individual cells into the solvent with higher dispersion efficiency.

In the cell analysis method according to the present embodiment, a sample with the water-insoluble embedding medium having been removed is heated in the presence of a divalent carboxylic acid compound, accordingly making it possible to separate and disperse individual cells into the solvent with higher dispersion efficiency. This can minimize error in the analysis result, which would otherwise occur due to cell lumps contained in the sample analyzed using a flow cytometer. Thus, the cell analysis method according to the present embodiment is capable of high-precision analysis using a flow cytometer on cells from a tissue.

In the cell analysis method according to the present embodiment, first, a water-insoluble embedding medium is removed from a sample obtained from a tissue specimen which is aldehyde-fixed and embedded in the embedding medium. Thereby, the obtained sample contains the tissue and from which the embedding medium is removed [step (A)]. Performing step (A) makes it possible to store the original characteristics that cells have pertaining to a three-dimensional structure.

In the present embodiment, the terms "aldehyde" is not particularly limited, but is associated with compounds that are capable of fixing tissues. In the present embodiment, examples of aldehyde include formalin (also called formaldehyde), glutaraldehyde, and the like. In the present embodiment, the aldehyde is particularly preferably formalin.

In the present embodiment, the term "water-insoluble embedding medium" may be any well-known water-insoluble embedding medium that is used to enable specimen section preparation in a natural state, and is not particularly limited. In the present embodiment, examples of the water-insoluble embedding medium include paraffin, celloidin, carbowax, mixtures of at least two of these, and the like, but are not particularly limited thereto. In the present embodiment, the water-insoluble embedding medium is preferably paraffin.

In the present embodiment, examples of the tissue include tissues such as solid tumor tissues (tumor tissues), non-tumor diseased tissues, and normal tissues.

Herein, the term "solid tumor" refers to one forming a lump made of tumor cells, fibroblasts, extracellular matrix, and the like. Specific examples of the solid tumor include laryngeal cancer, esophageal cancer, stomach cancer, colon cancer, colorectal cancer, rectal cancer, small-cell lung cancer, non-small cell lung cancer, pancreatic cancer, kidney cancer, liver cancer, thymic carcinoma, splenic cancer, thyroid cancer, parathyroid cancer, adrenal cancer, prostate cancer, bladder cancer, ovarian cancer, endometrial cancer, cervical cancer, bone cancer, skin cancer, sarcoma, osteosarcoma, melanoma, blastoma, squamous-cell carcinoma, non-squamous-cell carcinoma, brain tumor, oral cancer, carcinoma, lymphoma, fibroma, meningioma, biliary tract cancer, pheochromocytoma, islet cell carcinoma, Li-Fraumeni tumor, pituitary tumor, multiple neuroendocrine neoplasia type 1 and type 2, head and neck cancer, testicular cancer, and the like, but are not particularly limited thereto. Herein, the term "non-tumor diseased tissue" refers to a diseased tissue other than tumors. Examples of the non-tumor diseased tissue include benign prostatic hyperplasia, oral leukoplakia, colorectal polyp, esophageal precancerous growth, benign lesion, and the like, but are not particularly limited thereto. The normal tissue refers to a tissue other than the tumor tissues and non-tumor diseased tissues, and examples thereof include tissues obtained from organs such as the stomach, liver, breast, mammary gland, lung, pancreas, pancreas gland, uterus, skin, esophagus, larynx, pharynx, tongue, and thyroid gland, and the like, but embodiments are not limited thereto.

The sample obtained from the tissue specimen which is aldehyde-fixed and embedded in the water-insoluble embedding medium is obtained by cutting the tissue specimen in such a manner that the sample has a width larger than the size of the cells to be analyzed contained in the tissue. Thus, the thickness of the section is preferably 40 to 100 μm, and more preferably 50 to 70 μm, from the viewpoint of precisely analyzing the original characteristics of the cells. The section of the tissue specimen can be prepared, for example, using a microtome or by other methods.

The embedding medium can be removed by any known method in accordance with the type of the embedding medium. For example, in the case where the embedding medium is paraffin, the removal treatment can be performed by incubating the paraffin-embedded tissue specimen in a deparaffinization agent. The deparaffinization agent is a reagent capable of dissolving the paraffin contained in the paraffin-embedded tissue specimen without damaging the tissue in the paraffin-embedded tissue specimen. Examples of the deparaffinization agent include organic solvents such as xylene, benzene, and toluene, but are not particularly limited thereto. In the case where the embedding medium is paraffin, the paraffin can be removed by passing the paraffin-embedded tissue specimen through a layer of the organic solvent. When the embedding medium is subjected to the removal treatment, the treatment period and the treatment temperature can be determined as appropriate in accordance with the type of the tissue to be analyzed.

Moreover, from the viewpoint of analyzing the cells more precisely, the section may be rehydrated (hydrophilic treatment) after the embedding medium is removed. For example, in the case where the embedding medium is paraffin, the section after the deparaffinization treatment may be rehydrated by, for example, incubation in a descending series of ethanol solutions (for example, 100 volume % ethanol, an aqueous solution of 90 volume % ethanol, an aqueous solution of 80 volume % ethanol, and an aqueous solution of 70 volume % ethanol in the order of use). Note that the descending series of ethanol solutions are not particularly limited, as long as the series are capable of rehydrating the section after the deparaffinization.

Next, the sample obtained in step (a) is heated in the presence of a divalent carboxylic acid compound. Thereby, obtained is the heat-treated sample containing the tissue [step (b)]. Proteins in and on the surface of the cells constituting the aldehyde-fixed tissue may have cross-links different from that of original proteins of the cells. Thus, in am embodiment, performing step (b) breaks the cross-link, enabling the state of the proteins constituting the cells to return to the original state, or the state of the proteins to be similar to the original state.

The heat treatment in step (b) can be performed by, for example, heating a container containing the sample obtained in step (a) and the divalent carboxylic acid compound at a predetermined temperature (heat treatment temperature) for a predetermined period (heat treatment period) in an incubator.

Herein, in the embodiment, the term "divalent carboxylic acid compound" includes divalent carboxylic acids, salts of the divalent carboxylic acids, and anhydrides of the divalent carboxylic acids. The divalent carboxylic acid compound is preferably a highly water-soluble divalent carboxylic acid compound from the viewpoint of ensuring the easiness of the operation after the heat treatment of step (b). Herein, the term "highly water-soluble" refers to the solubility of 0.1 g or more of the compound per 100 g of water at 20°C. The solubility in water at 20°C is 0.1 g or more, preferably 0.2 g or more, from the viewpoint of ensuring the easiness of the operation after the heat treatment in step (b). The highly water-soluble divalent carboxylic acid compound should be a divalent carboxylic acid compound having a solubility of 0.1 g or more in 100 g of water at 20°C, and examples thereof include a divalent carboxylic acid compound with 2 to 7 carbon atoms and the like. The number of carbon atoms in the divalent carboxylic acid compound with 2 to 7 carbon atoms is 7 or less, preferably 5 or less, and more preferably 4 or less, from the viewpoint of ensuring the easiness of the operation after the heat treatment in step (b). Specific examples of the divalent carboxylic acid compound with 2 to 7 carbon atoms include compounds represented by Formula (I):

$$HOOC\text{-}R^1\text{-}COOH \qquad (I)$$

where $R^1$ represents a direct bond to an alkylene group with 1 to 5 carbon atoms which may have a substituent, an alkenylene group with 2 to 5 carbon atoms which may have a substituent, an alkynylene group with 2 to 5 carbon atoms which may have a substituent), or the like.

In Formula (I), examples of the alkylene group with 1 to 5 carbon atoms include a methylene group, an ethylene group, a n-propylene group, an isopropylene group, a n-butylene group, a tert-butylene group, a n-pentylene group, and the like, but are not particularly limited thereto. Examples of the alkenylene group with 2 to 5 carbon atoms include: a vinylene group, a propenylene group, a butenylene group, a pentenylene group, and the like, but are not particularly limited thereto. Examples of the alkynylene group with 2 to 5 carbon atoms include, an ethynylene group, a propynylene group, a butynylene group, a pentynylene group, and the like, but are not particularly limited thereto. Examples of substituents which the alkylene group with 1 to 5 carbon atoms, the alkenylene group with 2 to 5 carbon atoms, and the alkynylene group with 2 to 5 carbon atoms may have include a hydroxyl group, an amino group, and the like, but are not particularly limited thereto. Note that in the case where the substituent is an amino group, the divalent carboxylic acid compound is desirably a compound, which does not have an amino group at the $\alpha$ position of the carboxyl group from the viewpoint of improving the cell dispersion efficiency.

The divalent carboxylic acid compound is preferably a divalent carboxylic acid compound with 2 to 7 carbon atoms, more preferably at least one selected from the group consisting of aspartic acid, oxalic acid, malic acid, citraconic acid, maleic acid, glutaric acid, and succinic acid, from the viewpoints of ensuring the easiness of the operation after the heat treatment in step (b) and analyzing the cells more precisely.

Step (b) is performed by heating the sample obtained in step (a) in a solution containing the divalent carboxylic acid compound. In the solution containing the divalent carboxylic acid compound, a solvent is preferably water containing no impurity, that is, pure water, more preferably distilled water, and further preferably deionized water. The heat treatment is preferably performed under an approximately neutral condition from the viewpoints of reflecting the original physiological function of the cells and analyzing the cells more precisely. Thus, the pH of the solution containing the divalent carboxylic acid compound is 5 or more, preferably 6 or more, and more preferably 7 or more, from the viewpoints of reflecting the original physiological function of the cells and analyzing the cells more precisely. From the same viewpoints, the pH is 9 or less, preferably 8 or less, and more preferably 7.5 or less. In addition, the pH of the solution containing the divalent carboxylic acid compound can be adjusted with an aqueous solution of sodium hydroxide or the like. The concentration of the divalent carboxylic acid compound in the solution containing the divalent carboxylic acid compound is 0.02 mg/mL or more, preferably 0.5 mg/mL or more, from the viewpoints of reflecting the original physiological function of the cells and analyzing the cells more precisely. From the same viewpoints, the concentration is 20 mg/mL or less, preferably 1 mg/mL or less.

In a case of performing the heat treatment under an atmospheric pressure condition, the lower limit of the heat treatment temperature is preferably 80°C or more, more preferably 90°C or more, from the viewpoints of reflecting the original physiological function of the cells and analyzing the cells more precisely. In this case, the upper limit of the heat treatment temperature is a temperature at which the solution containing the divalent carboxylic acid compound does not boil, 100°C or less, more preferably 98°C or less, from the same viewpoints. Incidentally, the heat treatment may be performed under a pressurized condition. In this case, the heat treatment can be performed at a heat treatment temperature higher than that in the case of performing the heat treatment under an atmospheric pressure condition.

The heat treatment period can be determined as appropriate in accordance with the type of the cells to be analyzed, the heat treatment temperature, and so forth. The heat treatment period is not particularly limited, but is usually 20 to 60 minutes.

Next, the sample obtained in step (b) is brought into contact with an enzyme having a cell dispersion activity to separate, into individual cells, cells contained in the sample obtained in step (b). Thereby, the obtained sample contains the individual cells dispersed in the solvent [step (c)].

Step (c) can be performed by, for example, incubating a container containing the sample obtained in step (b) and an enzyme solution containing the enzyme with a cell dispersion activity under conditions (reaction temperature, reaction period) suitable for a reaction of the enzyme in an incubator or the like.

Herein, the term "cell dispersion activity" refers to an enzyme activity to separate cells adhering to or aggregating with each other in the tissue contained in the sample obtained in step (b). Examples of an enzyme with the cell dispersion activity include collagenase, dispase, and the like, but are not particularly limited thereto. These enzymes may be used alone, or may be used in a mixture of two or more. The enzyme with such a cell dispersion activity is preferably collagenase and dispase from the viewpoint of efficiently separating the cells from the tissue.

The reaction temperature can be determined as appropriate in accordance with the type of enzyme, the amount of enzyme used, the volume of tissue contained in the heat-treated sample, and so forth. From the viewpoints of allowing the enzyme to act sufficiently and maintaining the physiological function of the cells, the reaction temperature is usually a temperature close to a temperature *in vivo,* for example, 32 to 38°C, preferably 36 to 37.5°C, and more preferably around 37°C (for example, 37°C $\pm$ 0.2°C).

The reaction period can be determined as appropriate in accordance with the type of enzyme, the amount of enzyme used, the volume of tissue contained in the heat-treated sample, the time allowed to be consumed for the cell analysis, and so forth. The reaction period is usually 15 to 60 minutes, preferably 20 to 30 minutes, and more preferably around 20 minutes (for example, 20 $\pm$ 2 minutes).

In step (c), the enzyme solution can be used in an amount sufficient to immerse the tissue contained in the sample obtained in step (b). In this case, the enzyme concentration in the enzyme solution can be determined as appropriate in accordance with the volume of the tissue contained in the heat-treated sample, the type of the enzyme, and so forth. More specifically, for example, in the case of using collagenase as the enzyme, the collagenase concentration in the enzyme solution is preferably 25 units/mL or more, more preferably 30 units/mL or more, from the viewpoint of shortening the time required for the cell analysis; meanwhile, from the viewpoint of reducing the amount of the enzyme used, the collagenase concentration is preferably 1300 units/mL or less, more preferably 1250 units/mL or less. Here, 1 unit of collagenase refers to the amount of enzyme lysing 1 $\mu$g of a substrate 4-phenyl-azobenzyl-oxycarbonyl-Pro-Leu-Gly-Pro-D-Arg (SEQ ID NO: 1) at 25°C in a reaction system at a pH of 7.4. In the case of using dispase as the enzyme, the dispase concentration in the enzyme solution is 3 units/mL or more, preferably 4 units/mL or more, from the viewpoint of shortening the time required for the cell analysis. Meanwhile, from the viewpoint of reducing the amount of the enzyme used, the dispase concentration is 200 units/mL or less, more preferably 190 units/mL or less. Here, 1 unit of dispase refers to the amount of the enzyme that releases folin-positive amino acids from casein equivalent to 1 $\mu$mol of tyrosine at 37°C per minute in a reaction system at a pH of 7.5. In addition, the amount of the enzyme per 1 $mm^3$ of cells is usually preferably 3 to 8.5 units. Note that in the case of using a combination of collagenase and dispase as the enzyme, collagenase/dispase (unit ratio per unit amount) is usually preferably 4 to 10, more preferably 6.5 to 7.5. Moreover, in the case of using a combination of collagenase and dispase as the enzyme, a mixture of the two may be added to the heat-treated sample, or the two may be added separately to the heat-treated sample.

It should be noted that the cells contained in the sample obtained in this step (c) can be labeled with an appropriate labeling substance in accordance with an analysis use, the type of a laser light source of a flow cytometer to be described later, and so forth. Examples of the labeling substance include fluorescent substances such as 4',6-diamidino-2'-phenylindole dihydrochloride (DAPI), Hoechst 33342, propidium iodide (PI), pyronin Y, 7-amino-actinomycin D (7-AAD), fluorescein isothiocyanate (FITC), phycoerythrin (PE), allophycocyanin (APC), Texas Red (TR), Cy3, Cy5, PerCO(registered trademark) (manufactured by BD Bioscience), Alexa Fluor 405, Alexa Fluor 488, and Alexa Fluor 647(registered trademark) (manufactured by Life Technologies Corporation), but are not particularly limited thereto. Further, the cells may be labeled using a compound containing a labeling substance and a binding substance capable of specifically binding to a marker molecule or the like in accordance with the analysis use, or a complex formed of a labeling substance and a binding substance.

Next, optical information on the cells in the sample obtained in step (c) is obtained using the flow cytometer [step (d)]. Specifically, the sample obtained in step (c) is supplied to a flow cell of the flow cytometer. Then, the sample supplied to the flow cell is irradiated with light from a light source of the flow cytometer. Subsequently, optical information on the cells in the sample irradiated with the light is detected with a detector of the flow cytometer. In this way, the optical information on the cells in the sample obtained in step (c) is obtained using the flow cytometer.

The optical information includes at least one selected from the group consisting of image information, scattered light information, and fluorescence information. The image information includes reflected light image information, transmitted light image information, and fluorescence image information. The scatted light information includes forward scattered light information reflecting the size of cells and side scattered light information reflecting the surface complexity of cells. The fluorescence information is information obtained from fluorescence emitted from the labeling substance used. The fluorescence information is information obtained by irradiating a measurement sample with light at an excitation wavelength corresponding to the labeling substance, and then measuring fluorescence from the labeling substance. Specific examples of the fluorescence information include a fluorescence intensity (measurement value) detected from the fluorescence emitted by the labeling substance, a maximum value of the fluorescence intensity, an integral value of the fluorescence intensity, a fluorescence pulse width, and the like, but are not particularly limited thereto.

In step (d), the cell concentration in the sample obtained in step (c) should be a concentration at which individual cells among the cells can be electrochemically identified and detected by flow cytometry. The cell concentration in the sample obtained in step (c) is not particularly limited, but is usually $1\times10^6$ to $1\times10^7$ cells/mL.

In obtaining optical information using the flow cytometer, the flow rate of the sample introduced into the flow cell should be at rate at which individual cells among the cells can be electrochemically identified and detected by flow cytometry. The flow rate of the sample introduced into the flow cell is not particularly limited, but is usually 10 to 120 $\mu$L/min.

Then, the cells are analyzed based on the optical information obtained in step (d) [step (e)].

The cells can be analyzed by, for example, creating a scattergram based on the optical information, and analyzing the scattergram. A scattergram can be created in accordance with the analysis purpose. Examples of such a scattergram include a scattergram with two axes of fluorescence information and forward scattered light information, a scattergram with two axes of forward scattered light information and side scattered light information, and the like. Such a scattergram can be analyzed by, for example, causing a computer to execute a process including: (S-1) a step of specifying, in the scattergram, a border of region A where cells A with a predetermined nature are detected; and (S-2) a step of counting the number of cells included in a cell population appearing in region A, and the number of cells appearing outside region A. Thus, the cell analysis method according to the present embodiment can provide, for example, quantitative population information on cells constituting a tumor tissue, a non-tumor diseased tissue, or the like.

Examples

Hereinafter, the invention is described in detail by use of Examples and so forth. However, the invention is not limited to these. Note that solubilities in 100 g of water at 20°C of compounds used below are as follows.

<Solubilities in 100 g of water at 20°C>

| | |
|---|---|
| oxalic acid: | 10.2 g |
| glutaric acid: | 43 g |
| malic acid: | 5 g |
| succinic acid: | 8 g |
| maleic acid: | 44 g |
| citraconic acid: | 10 g or more |
| aspartic acid: | 0.45 g |
| citraconic anhydride: | 10 g or more |
| acetic acid: | 100 g |
| 2-hydroxybutyric acid: | 5 g or more |
| 3-methylcrotonic acid: | 2 g or more |
| aminobutanoic acid: | 5 g or more |
| citrulline: | 20 g |

Examples 1 to 7 and Comparative Examples 1 to 5

(1) Preparation of Sample from FFPE Tissue Specimen for Examining Dispersion Efficiency

As a FFPE tissue specimen, used is a FFPE tissue specimen (5 mm×5 mm×5 mm) of a tissue containing adenocarcinomic human alveolar basal epithelial cells, A549 cells, in a cancer-bearing mouse bearing the cancer cells. The FFPE tissue specimen is sliced to a thickness of 50 $\mu$m to obtain a thin section (5 mm×5 mm×50 $\mu$m).

The obtained thin section is incubated in xylene at 25°C for 5 minutes. This incubation is repeated twice, thereby removing paraffin from the thin section (removal treatment of the embedding medium). Then, the section after the removal treatment of the embedding medium is incubated at 5°C for 3 minutes in a descending series of ethanol solutions (100 volume % ethanol, an aqueous solution of 90 volume % ethanol, an aqueous solution of 80 volume % ethanol, and an aqueous solution of 70 volume % ethanol in the order of use). Thus, the section after the removal treatment of the embedding medium is rehydrated (hydrophilic treatment).

Subsequently, the section after this hydrophilic treatment is incubated in any one of the following test samples at 98°C for 20 minutes (heat treatment). Note that the test samples used are: an aqueous solution of 0.1 mass % oxalic acid (pH 7.3, Example 1), an aqueous solution of 0.1 mass % glutaric acid (pH 7.4, Example 2), an aqueous solution of 0.1 mass % malic acid (pH 7.2, Example 3), an aqueous solution of 0.1 mass % succinic acid (pH 7.4, Example 4), an aqueous solution of 0.1 mass % maleic acid (pH 7.3, Example 5), an aqueous solution of 0.1 mass % citraconic acid (pH 7.4, Example 6), an aqueous solution of 0.1 mass % aspartic acid (pH 7, Example 7), an aqueous solution of 0.1 mass % acetic acid (pH 7.4, Comparative Example 1), an aqueous solution of 0.1 mass % 2-hydroxybutyric acid (pH 7.4, Comparative Example 2), an aqueous solution of 0.1 mass % 3-methylcrotonic acid (pH 7.4, Comparative Example 3), an aqueous solution of 0.1 mass % aminobutanoic acid (pH 7.4, Comparative Example 4), and an aqueous solution of 0.1 mass % citrulline (pH 7.3, Comparative Example 5).

The section after heat treatment, is incubated at 37°C for 20 minutes in an enzyme solution [phosphate-buffered saline (PBS), Hanks' balanced salt solution (HBSS), or culture solution for culturing human cell line (all have a pH of 7.4 and contain 125 U/mL of collagenase and 18.28 U/mL of dispase)]. The resulting mixture is lightly stirred by pipetting to promote the separation and dispersion of the cells from the section. Thus, a sample is obtained for examining the dispersion efficiency. Meanwhile, as a control, a sample for examining the dispersion efficiency is obtained by conducting the same procedure as above, except that an aqueous solution of 0.1 mass % citric acid (pH 6) is used in place of the test samples of Examples 1 to 7 and Comparative Examples 1 to 5.

(2) Examination of Dispersion Efficiency

The nuclei of the cells in each of the obtained samples are stained with Hoechst 33342, and then observed with a fluorescence microscope to count the number of cell groups (a total of single cells and cell lumps), the number of the single cells, and the number of the cell lumps. Note that a lump observed to have two or more stained nuclei overlapping with each other is designated a "cell lump," while one having a single stained nucleus is designated a "single cell." Additionally, a dispersion efficiency is calculated as a ratio of single cells in cell groups according to Formula (A):

[Math. 1]

$$\text{Dispersion Efficiency (\%)} = (\text{number of single cells})/(\text{number of cell groups}). \quad (A)$$

Next, a ratio of dispersion efficiency obtained from a test sample to a dispersion efficiency obtained from an aqueous solution of citric acid (relative dispersion efficiency) is calculated according to Formula (B):

[Math. 2]

$$\text{Relative dispersion efficiency (\%)} = (\text{dispersion efficiency of a test}$$

$$\text{sample})/(\text{dispersion efficiency from use of an aqueous solution of citric acid}) \times 100$$

(B)

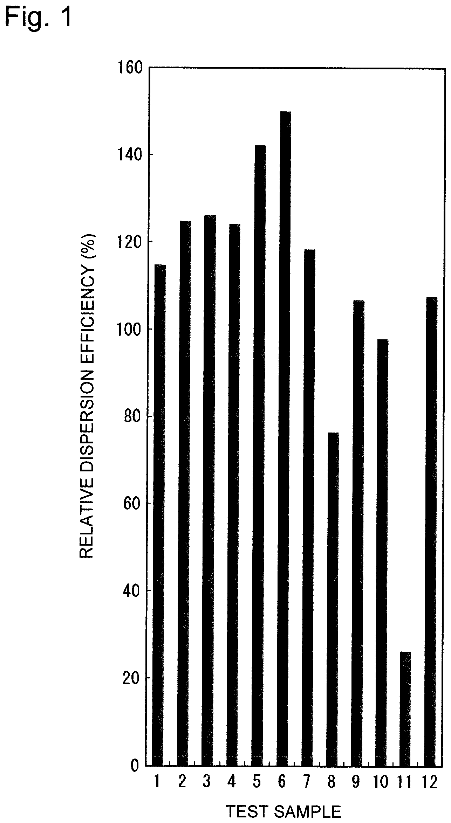

Fig. 1 illustrates the results of examining the relative dispersion efficiencies obtained by using the test samples of Examples 1 to 7 and Comparative Examples 1 to 5. In Fig. 1, test sample 1 represents the aqueous solution of 0.1 mass % oxalic acid (Example 1); test sample 2, the aqueous solution of 0.1 mass % glutaric acid (Example 2); test sample 3, the aqueous solution of 0.1 mass % malic acid (Example 3); test sample 4, the aqueous solution of 0.1 mass % succinic acid (Example 4); test sample 5, the aqueous solution of 0.1 mass % maleic acid (Example 5); test sample 6, the aqueous solution of 0.1 mass % citraconic acid (Example 6); test sample 7, the aqueous solution of 0.1 mass % aspartic acid (Example 7); test sample 8, the aqueous solution of 0.1 mass % acetic acid (Comparative Example 1); test sample 9, the aqueous solution of 0.1 mass % 2-hydroxybutyric acid (Comparative Example 2); test sample 10, the aqueous solution of 0.1 mass % 3-methylcrotonic acid (Comparative Example 3); test sample 11, the aqueous solution of 0.1 mass % aminobutanoic acid (Comparative Example 4); and test sample 12, the aqueous solution of 0.1 mass % citrulline (Comparative Example 5).

It can be seen from the results illustrated in Fig. 1 that when each of test samples 1 to 7 is used (Examples 1 to 7), the relative dispersion efficiency is 110% or more. This surprisingly reveals that when each of test samples 1 to 7 is used (Examples 1 to 7), the cell dispersion efficiency is higher than that obtained when the aqueous solution of citric acid is used. These results reveal that, in dispersing the cells of the FFPE tissue specimen, the heat treatment in the presence of oxalic acid, glutaric acid, malic acid, succinic acid, maleic acid, citraconic acid, or aspartic acid allows improved relative cell dispersion efficiency. In contrast, it was found that when test samples 8 to 12 (Comparative Examples 1 to 5) are used, the cell dispersion efficiencies are approximately the same as or lower than that obtained when the aqueous solution of citric acid is used.

Oxalic acid, glutaric acid, malic acid, succinic acid, maleic acid, citraconic acid, and aspartic acid are each a highly water-soluble (solubility in water at 20°C: 0.1 g or more) divalent carboxylic acid having two carboxyl groups. On the other hand, citric acid is a trivalent carboxylic acid having three carboxyl groups; meanwhile, acetic acid, 2-hydroxybutyric acid, 3-methylcrotonic acid, aminobutanoic acid, and citrulline are each a monovalent carboxylic acid having one carboxyl group. The above results reveal that, in dispersing the cells of the FFPE tissue specimen, heat treatment in the presence of a divalent carboxylic acid surprisingly allows higher dispersion efficiency of samples containing individual cells dispersed in a solvent.

Example 8 and Comparative Example 6

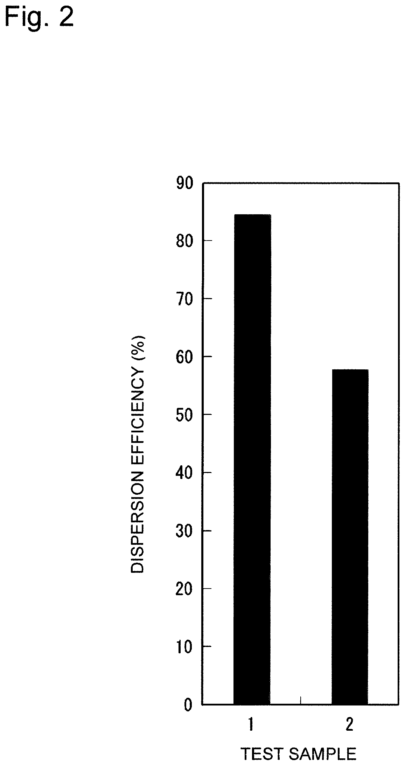

The same procedures as in (1) and (2) of Examples 1 to 7 and Comparative Examples 1 to 5 are conducted, except that an aqueous solution of 0.05 mass % citraconic acid (pH 7.4, Example 8) and an aqueous solution of 0.05 mass % citric acid (pH 6, Comparative Example 6) are used as test samples in (1) of Examples 1 to 7 and Comparative Examples 1 to 5 to obtain cell dispersion efficiencies. Fig. 2 illustrates the results of examining the cell dispersion efficiencies obtained by using the test samples of Example 8 and Comparative Example 6 (the aqueous solution of 0.05 mass % citraconic acid and the aqueous solution of 0.05 mass % citric acid). In Fig. 2, test sample 1 represents the aqueous solution of 0.05 mass % citraconic acid (Example 8). Test sample 2 represents the aqueous solution of 0.05 mass % citric acid (Comparative Example 6).

It can be seen from the results illustrated in Fig. 2 that when test sample 1 is used (Example 8), the dispersion efficiency is significantly higher than that obtained when test sample 2 is used (Comparative Example 6). These surprising results reveal that even if a divalent carboxylic acid is used at a low concentration in the heat treatment in dispersing the cells of the FFPE tissue specimen, it is possible to obtain a sample containing individual cells dispersed in a solvent with high dispersion efficiency.

Examples 9 to 11 and Comparative Example 7

Samples for flow cytometry analysis are obtained by conducting the same procedure as in (1) of Examples 1 to 7 and Comparative Examples 1 to 5, except that the heat treatment is performed using any one of test samples of an aqueous solution of 0.1 mass % maleic acid (pH 7.3, Example 9), an aqueous solution of 0.1 mass % citraconic acid (pH 7.4, Example 10), an aqueous solution of 0.1 mass % citraconic anhydride (pH 7.4, Example 11), and an aqueous solution of 0.1 mass % citric acid (pH 6, Comparative Example 7) in (1) of Examples 1 to 7 and Comparative Examples 1 to 5.

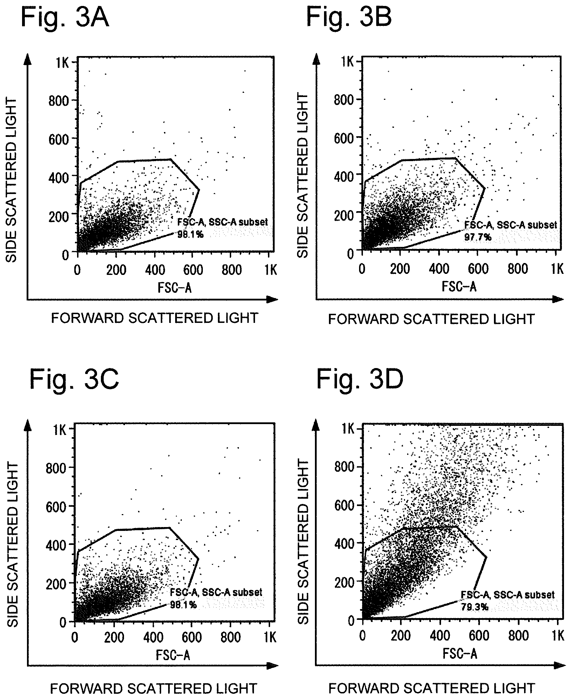

Forward scattered light and side scattered light from the cells in the obtained samples for flow cytometry analysis are measured using a flow cytometer to obtain scattergrams. Figs. 3A to 3D illustrate the scattergrams with parameters of forward scattered light intensity and side scattered light intensity obtained by performing the heat treatment using the aqueous solution of 0.1 mass % maleic acid (Example 9), the aqueous solution of 0.1 mass % citraconic acid (Example 10), the aqueous solution of 0.1 mass % citraconic anhydride (Example 11), and the aqueous solution of 0.1 mass % citric acid (Comparative Example 7). Fig. 3A is a scattergram obtained by using the aqueous solution of 0.1 mass % maleic acid (Example 9). Fig. 3B is a scattergram obtained by using the aqueous solution of 0.1 mass % citraconic acid (Example 10). Fig. 3C is a scattergram obtained by using the aqueous solution of 0.1 mass % citraconic anhydride (Example 11). Fig. 3D is a scattergram obtained by using the aqueous solution of 0.1 mass % citric acid (Comparative Example 7).

Generally, forward scattered light indicates the size of cells, and side scattered light indicates the surface complexity of cells. It can be seen from the results illustrated in Figs. 3A to 3D that most of plots corresponding to the cells are observed within the frames in the scattergrams obtained by using the aqueous solution of 0.1 mass % maleic acid [Fig. 3A)], the aqueous solution of 0.1 mass % citraconic acid [Fig. 3B], and the aqueous solution of 0.1 mass % citraconic anhydride [Fig. 3C]. These surprising results reveal that the heat treatment in the presence of any one of maleic acid, citraconic acid, and citraconic anhydride makes is possible to obtain a sample containing cells having a uniform size and a uniform surface state (i.e., single cells) dispersed in a solvent with high dispersion efficiency. In contrast, it can be seen from the scattergram obtained by using the aqueous solution of 0.1 mass % citric acid (Comparative Example 7) that plots corresponding to the cells are observed in a wider area. This surprising result reveals that a large number of cell lumps remain in the resulting sample by the heat treatment in the presence of citric acid.

When the sample containing cells having a uniform size and a uniform surface state (i.e., single cells) dispersed in a solvent with high dispersion efficiency is used as a sample for flow cytometry analysis, it is possible to minimize error in the analysis result, which would otherwise occur due to contamination of optical information derived from cell lumps. Thus, it is suggested that, in dispersing cells of a FFPE tissue specimen, heat treatment in the presence of a divalent carboxylic acid enables high-precision analysis using a flow cytometer on the cells contained in the tissue.

[Sequence Listing Free Text]

SEQ ID NO: 1 is a sequence of a collagenase substrate. Xaa at position 1 is a 4-phenyl-azobenzyl-oxycarbonyl-proline residue.

As has been described above, the cell analysis method according to embodiments enable a cell analysis method capable of high-precision analysis using a flow cytometer on cells from a tissue.

## Claims

1. A cell analysis method for analyzing, using a flow cytometer, cells obtained from a tissue specimen which is aldehyde-fixed and embedded in a water-insoluble embedding medium, the method comprising:

   (a) removing embedding medium from a sample obtained from the tissue specimen which is aldehyde-fixed and embedded in the water-insoluble embedding medium, thereby obtaining the sample comprising the tissue from which the embedding medium is removed;
   (b) heating the sample obtained from step (a) in the presence of a divalent carboxylic acid compound to obtain a heat-treated sample comprising the tissue;
   (c) contacting the heat-treated sample from step (b) with an enzyme having a cell dispersion activity to separate the sample into individual cells, thereby obtaining the sample comprising the individual cells dispersed in a solvent;
   (d) subjecting the separated cells from step (c) to flow cytometry to obtain optical information ; and
   (e) analyzing the cells based on the optical information obtained in the step (d).

2. The method according to claim 1, wherein, step (b) comprises heating the sample from step (a) in a solution containing the divalent carboxylic acid compound.

3. The method according to claim 2, wherein the solution containing the divalent carboxylic acid compound has a pH of 5 to 9.

4. The method according to claim 2 or 3, wherein a concentration of the divalent carboxylic acid compound in the solution containing the divalent carboxylic acid compound is 0.5 to 10 mg/mL.

5. The method according to any one of claims 1 to 4, wherein the divalent carboxylic acid compound has 2 to 7 carbon atoms.

6. The method according to any one of claims 1 to 5, wherein the solubility of the divalent carboxylic acid compound is 0.1 g or more in 100 g of water at 20°C.

7. The method according to any one of claims 1 to 6, wherein the divalent carboxylic acid compound comprises at least one divalent carboxylic acid selected from the group consisting of aspartic acid, oxalic acid, malic acid, citraconic acid, maleic acid, glutaric acid, and succinic acid.

8. The method according to any one of claims 1 to 7, wherein the enzyme is at least one selected from the group consisting of collagenase and dispase.

9. The method according to any one of claims 1 to 8, wherein the aldehyde is formalin.

10. The method according to any one of claims 1 to 9, wherein the water-insoluble embedding medium is paraffin.

11. The method according to any one of claims 1 to 10, wherein the sample obtained from the tissue specimen which is aldehyde-fixed and embedded in the water-insoluble embedding medium is a section with a thickness of 50 to 100 µm.

12. The method according to any one of claims 1 to 11, wherein the optical information is at least one of image information, scattered light information, and fluorescence information.

13. The method according to claim 12, wherein the scattered light information is forward scattered light information and side scattered light information.


**Patentansprüche**

1. Zellanalyseverfahren zum Analysieren, unter Verwendung eines Durchflusszytometers, von Zellen, erhalten aus einem Gewebeprobestück, welches Aldehyd-fixiert und in ein Wasser-unlösliches Einbettungsmedium eingebettet ist, wobei das Verfahren umfasst:

(a) Entfernen von Einbettungsmedium aus einer Probe, erhalten aus dem Gewebeprobestück, welches Aldehyd-fixiert und in das Wasser-unlösliche Einbettungsmedium eingebettet ist, wodurch die Probe erhalten wird, welche das Gewebe umfasst, von dem das Einbettungsmedium entfernt ist;
(b) Erhitzen der aus Schritt (a) erhaltenen Probe in der Gegenwart einer divalenten Carboxylsäure-Verbindung, um eine Hitze-behandelte Probe zu erhalten, welche das Gewebe umfasst;
(c) Inkontaktbringen der Hitze-behandelten Probe aus Schritt (b) mit einem Enzym mit einer Zelldispersionsaktivität, um die Probe in einzelne Zellen zu trennen, wodurch die Probe erhalten wird, welche die einzelnen in einem Lösungsmittel dispergierten Zellen umfasst;
(d) Durchführen von Durchflusszytometrie mit den getrennten Zellen aus Schritt (c), um optische Information zu erhalten; und
(e) Analysieren der Zellen, basierend auf der im Schritt (d) erhaltenen optischen Information.

2. Verfahren gemäß Anspruch 1, wobei Schritt (b) das Erhitzen der Probe aus Schritt (a) in einer Lösung, welche die divalente Carboxylsäure-Verbindung enthält, umfasst.

3. Verfahren gemäß Anspruch 2, wobei die Lösung, welche die divalente Carboxylsäure-Verbindung enthält, einen pH-Wert von 5 bis 9 aufweist.

4. Verfahren gemäß Anspruch 2 oder 3, wobei eine Konzentration der divalenten Carboxylsäure-Verbindung in der Lösung, welche die divalente Carboxylsäure-Verbindung enthält, 0,5 bis 10 mg/mL ist.

5. Verfahren gemäß einem der Ansprüche 1 bis 4, wobei die divalente Carboxylsäure-Verbindung 2 bis 7 Kohlenstoffatome aufweist.

6. Verfahren gemäß einem der Ansprüche 1 bis 5, wobei die Löslichkeit der divalenten Carboxylsäure-Verbindung 0,1 g oder mehr in 100 g Wasser bei 20°C ist.

7. Verfahren gemäß einem der Ansprüche 1 bis 6, wobei die divalente Carboxylsäure-Verbindung wenigstens eine divalente Carboxylsäure umfasst, ausgewählt aus der Gruppe, bestehend aus Asparaginsäure, Oxalsäure, Äpfelsäure, Citraconsäure, Maleinsäure, Glutarsäure und Bernsteinsäure.

8. Verfahren gemäß einem der Ansprüche 1 bis 7, wobei das Enzym wenigsten eines ist, ausgewählt aus der Gruppe, bestehend aus Kollagenase und Dispase.

9. Verfahren gemäß einem der Ansprüche 1 bis 8, wobei das Aldehyd Formalin ist.

10. Verfahren gemäß einem der Ansprüche 1 bis 9, wobei das Wasser-unlösliche Einbettungsmedium Paraffin ist.

11. Verfahren gemäß einem der Ansprüche 1 bis 10, wobei die Probe, erhalten aus dem Gewebeprobestück, welches Aldehyd-fixiert und in das Wasser-unlösliche Einbettungsmedium eingebettet ist, ein Schnitt mit einer Dicke von 50 bis 100 µm ist.

**12.** Verfahren gemäß einem der Ansprüche 1 bis 11, wobei die optische Information wenigstens eine aus Bildinformation, Streulichtinformation und Fluoreszenzinformation ist.

**13.** Verfahren gemäß Anspruch 12, wobei die Streulichtinformation Vorwärtsstreulichtinformation und Seitwärtsstreulichtinformation ist.

**Revendications**

**1.** Procédé d'analyse de cellules pour analyser, en utilisant un cytomètre en flux, des cellules obtenues à partir d'un spécimen de tissu qui est fixé par aldéhyde et enrobé dans un milieu d'enrobage insoluble dans l'eau, le procédé comprenant :

(a) l'élimination du milieu d'enrobage à partir d'un échantillon obtenu à partir du spécimen de tissu qui est fixé par aldéhyde et enrobé dans le milieu d'enrobage insoluble dans l'eau, pour ainsi obtenir l'échantillon comprenant le tissu duquel le milieu d'enrobage est éliminé ;
(b) le chauffage de l'échantillon obtenu issu de l'étape (a) en présence d'un composé d'acide carboxylique divalent pour obtenir un échantillon traité thermiquement comprenant le tissu ;
(c) la mise en contact de l'échantillon traité thermiquement issu de l'étape (b) avec une enzyme présentant une activité de dispersion de cellules pour séparer l'échantillon en cellules individuelles, pour ainsi obtenir l'échantillon comprenant les cellules individuelles dispersées dans un solvant ;
(d) la soumission des cellules séparées issues de l'étape (c) à une cytométrie en flux pour obtenir des informations optiques ; et
(e) l'analyse des cellules sur la base des informations optiques obtenues dans l'étape (d).

**2.** Procédé selon la revendication 1, dans lequel l'étape (b) comprend le chauffage de l'échantillon issu de l'étape (a) dans une solution contenant le composé d'acide carboxylique divalent.

**3.** Procédé selon la revendication 2, dans lequel la solution contenant le composé d'acide carboxylique divalent présente un pH de 5 à 9.

**4.** Procédé selon la revendication 2 ou 3, dans lequel une concentration du composé d'acide carboxylique divalent dans la solution contenant le composé d'acide carboxylique divalent est de 0,5 à 10 mg/ml.

**5.** Procédé selon l'une quelconque des revendications 1 à 4, dans lequel le composé d'acide carboxylique divalent contient 2 à 7 atomes de carbone.

**6.** Procédé selon l'une quelconque des revendications 1 à 5, dans lequel la solubilité du composé d'acide carboxylique divalent est de 0,1 g ou plus dans 100 g d'eau à 20 °C.

**7.** Procédé selon l'une quelconque des revendications 1 à 6, dans lequel le composé d'acide carboxylique divalent comprend au moins un acide carboxylique divalent sélectionné dans le groupe constitué de l'acide aspartique, de l'acide oxalique, de l'acide malique, de l'acide citraconique, de l'acide maléique, de l'acide glutarique, et de l'acide succinique.

**8.** Procédé selon l'une quelconque des revendications 1 à 7, dans lequel l'enzyme est au moins une sélectionnée dans le groupe constitué d'une collagénase et d'une dispase.

**9.** Procédé selon l'une quelconque des revendications 1 à 8, dans lequel l'aldéhyde est le formol.

**10.** Procédé selon l'une quelconque des revendications 1 à 9, dans lequel le milieu d'enrobage insoluble dans l'eau est une paraffine.

**11.** Procédé selon l'une quelconque des revendications 1 à 10, dans lequel l'échantillon obtenu à partir du spécimen de tissu qui est fixé par aldéhyde et enrobé dans le milieu d'enrobage insoluble dans l'eau est une section ayant une épaisseur de 50 à 100 $\mu$m.

**12.** Procédé selon l'une quelconque des revendications 1 à 11, dans lequel les informations optiques sont au moins une parmi des informations d'image, des informations de lumière diffusée, et des informations de fluorescence.

13. Procédé selon la revendication 12, dans lequel les informations de lumière diffusée sont des informations de lumière diffusée vers l'avant et des informations de lumière diffusée sur le côté.

# Fig. 1

Fig. 2

# Fig. 3A

FORWARD SCATTERED LIGHT

# Fig. 3B

FORWARD SCATTERED LIGHT

# Fig. 3C

FORWARD SCATTERED LIGHT

# Fig. 3D

FORWARD SCATTERED LIGHT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WILLEM E CORVER et al.** High-resolution multi-parameter DNA flow cytometry enables detection of tumour and stromal cell subpopulations in paraffin-embedded tissues. *The Journal of Pathology,* 11 April 2005, vol. 206, 233-241, http://interscience.wiley.com